# EUROPEAN PATENT SPECIFICATION

(11) **EP 1 807 068 B1**
(45) Date of publication and mention of the grant of the patent: **17.03.2010**
(21) Application number: 05821186.3
(22) Date of filing: 26.10.2005
(51) Int. Cl.: A61K 31/167, A61K 31/616, A61K 31/522, A61K 47/02, A61P 29/00

(54) **COMPOSITION COMPRISING ACETAMINOPHEN, CAFFEINE AND ASPIRIN TOGETHER WITH AN ALKILINE AGENT FR ENHANCED ABSORPTION**
ZUSAMMENSETZUNG MIT ACETAMINOPHEN, KOFFEIN UND ASPIRIN ZUSAMMEN MIT EINEM ALKALIWIRKSTOFF FÜR VERBESSERTE ABSORPTION
PRÉPARATION INCLUANT DE L ACÉTAMINOPHÈNE, DE LA CAFÉINE ET DE L ASPIRINE AVEC UN AGENT ALCALIN POUR EN FACILITER L ABSORPTION

(30) Priority: 28.10.2004 US 622812 P
(43) Date of publication of application: 18.07.2007
(73) Proprietor: Novartis AG, 4056 Basel (CH)
(72) Inventor: LIU, Rong, Warren, New Jersey 07059 (US); CORBO, Michael, Flemington, New Jersey 08822 (US); DESAI, Jatin, Plainsboro, New Jersey 08536 (US); FRUNZI, Gerard, P., Douglaston, New York 11362 (US); QI, Xiaohong, Irvington, New Jersey 07111 (US); CHOI, Candice, Y., Palisades Park, New Jersey 07650 (US); GANDHI, Rahul, R., Plainsboro, New Jersey 08536 (US); BOULOS, Atef, Z., Rockaway, New Jersey 07866 (US)
(74) Representative: Liphardt, Bernd
(86) International application number: PCT/US2005/038507
(87) International publication number: WO 2006/049978

(56) References cited:
- DE-A1- 19 502 789
- GB-A- 2 103 087
- US-A- 4 294 819
- US-A- 5 296 241
- US-B1- 6 316 025
- ROSTAMI-HODJEGAN A ET AL: "A NEW RAPIDLY ABSORBED PARACETAMOL TABLET CONTAINING SODIUM BICARBONATE. I. A FOUR-WAY CROSSOVER STUDY TO COMPARE THE CONCENTRATION-TIME PROFILE OF PARACETAMOL FROM THE NEW PARACETAMOL/SODIUM BICARBONATE TABLET AND A CONVENTIONAL PARACETAMOL TABLET IN FE" DRUG DEVELOPMENT AND INDUSTRIAL PHARMACY, NEW YORK, NY, US, vol. 28, no. 5, May 2002 (2002-05), pages 523-531, XP001097703 ISSN: 0363-9045 cited in the application
- GRATTAN T ET AL: "A five way crossover human volunteer study to compare the pharmacokinetics of paracetamol following oral administration of two commercially available paracetamol tablets and three development tablets containing paracetamol in combination with sodium bicarbonate or calcium carbonate." EUROPEAN JOURNAL OF PHARMACEUTICS AND BIOPHARMACEUTICS : OFFICIAL JOURNAL OF ARBEITSGEMEINSCHAFT FÜR PHARMAZEUTISCHE VERFAHRENSTECHNIK E.V. MAY 2000, vol. 49, no. 3, May 2000 (2000-05), pages 225-229, XP002367457 ISSN: 0939-6411 cited in the application

## Description

### Background of the Invention

Aspirin and acetaminophen (also referred to as paracetamol and APAP, respectively) are well-known analgesic and antipyretic agents. They are often employed with caffeine.

Buffered aspirin products that are formulated to simultaneously deliver alkaline material and aspirin to the stomach are known in the art. The alkaline material is co-administered with aspirin to reduce the acidity of the stomach content and to react with the aspirin to form a soluble salt thereof.

In the co-administration of tablets containing alkaline material(s) and aspirin, it is customary to separate the alkaline material(s) from the aspirin. One way to accomplish this is through the formulation of a multi-layer tablet in which the alkaline material(s) is (are) contained in one layer and the aspirin in another layer.

U.S. Patent 4,664,915 ('915 patent) discloses that in multi-layered compressed tablets the rate of reaction of the alkaline material with the acid content of the stomach can be increased if the tablets contain citric acid and monobasic sodium phosphate as part of the alkaline layer. As is evident from Claim 1 of the '915 patent, the alkaline component is selected from the group consisting of calcium carbonate, magnesium carbonate, a magnesium-oxy component and mixtures thereof. The magnesium-oxy component is selected from the group consisting of magnesium oxide, magnesium hydroxide, and combinations thereof.

Grattan et al., "A five way crossover human volunteer study to compares the pharmacokinetics of paracetamol following oral administration of two commercially available paracetamol tablets and three development tablets containing paracetamol in combination with sodium bicarbonate or calcium bicarbonate", Eur. J. Pharm Biopharm. 2000, 43 (3), 225-229), report that, in a panel of 15 fasted volunteers, paracetamol was absorbed faster from tablets containing 630 mg sodium bicarbonate than from conventional paracetamol tablets.

Rostami-Hodjegan et al., Drug Development and Industrial Pharmacy, 28 (5), 523-531 (2002)), report that, as indicated by a shorter tₘₐₓ in both the fed and fasted state and a higher Cₘₐₓ in the fasted state, as compared to conventional tablets, paracetamol is absorbed more rapidly from the tablets containing sodium bicarbonate. The two formulations were deemed bio-equivalent with respect to area under the curve (AUC). As indicated by AUC, food did not affect the extent of absorption from either formulation. However, as indicated by the longer Tₘₐₓ and Cₘₐₓ, food reduced the rate of absorption from both formulations.

Rostami-Hodjegan et al. refer to the work of Neilsen et al., "Analgesic Efficacy of Immediate and Sustained Paracetemol and Plasma Concentration of Paracetamol, Double Blind, Placebo Controlled Evaluation Using Painful Laser Stimulation", Eur. J. Clin. Pharmacol. 1992, 42, 261-264, who postulate that the rate of increase in paracetamol plasma concentration may be important in the alleviation of acute (laser-induced) pain.

Rostami-Hodjegan et al. also refer to Luthy et al., "The Analgesic Effect of Paracetamol Depends on Its Method of Administration" Scweiz. Med. Wochenschr. 1993, 123 (50/II), 406), who conclude that only rapid administration of paracetamol produces sufficiently high plasma levels at the peak to induce effective passage of the drug to the central nervous system and cause a significant analgesic effect.

After reviewing the relevant literature, Rostami-Hodjegan et al. opine that it appears likely that the faster rate of paracetamol absorption from paracetamol/sodium bicarbonate tablets would bring the clinical benefit of a faster onset of analgesic action and possibly a greater peak analgesic effect.

Nayak et al., Journal of Pharmaco Kinetics and Biopharmaceutics (1997) 5 (6), 597-613, studied the in-vitro dissolution profile, in-vitro and in-vivo buffering characteristics, and single dose bioavailability, of various buffered aspirin tablet formulations. Buffering agents, such as magnesium and aluminum hydroxides or magnesium carbonate and aluminum glycinate, were found to significantly increase the rate of aspirin dissolution from solid dosage forms, as compared to an unbuffered tablet formulation. The extent of aspirin absorption was equivalent with all formulations; however, the faster rate of dissolution (t50 and t90) with buffered formulations resulted in earlier and higher peak concentration of salicylate, as compared to that obtained with the unbuffered formulation.

Grattan et al., European Journal of Pharmaceutics and Biopharmaceutics (2000 May), 49 (3), 225-9, carried out a five-way crossover human volunteer study to compare the pharmacokinetics of paracetamol following oral administration of two commercially available paracetamol tablets and three development tablets containing paracetamol in combination with sodium bicarbonate or calcium carbonate. The results demonstrated that the addition of 630 mg of sodium bicarbonate to paracetamol tablets increased the rate of absorption of paracetamol relative to conventional paracetamol tablets and soluble paracetamol tablets. The addition of 400 mg of sodium bicarbonate to paracetamol tablets increased the absorption rate of paracetamol relative to conventional paracetamol tablets but there was no difference in the rate of absorption compared to soluble paracetamol tablets. Grattan et al. report that, compared to the conventional paracetamol tablet, the addition of 375 mg calcium carbonate to paracetamol tablets had no effect on absorption kinetics. Grattan et al. postulate that the faster absorption observed for the sodium bicarbonate formulations could be the result of an increase in gastric emptying rate leading to faster transport of paracetamol to the small intestine where absorption takes place.

Since, as compared to the conventional paracetamol tablet, the addition of a calcium salt, for example, calcium carbonate, to paracetamol tablets had no effect on absorption kinetics, one skilled in the art would be lead away from the use of a calcium salt. The skilled artisan would instead employ a sodium salt to increase the rate of absorption of paracetamol in paracetamol tablets.

Kelly et al., Pharmaceutical Research (2003), 20 (10), 1668-1673), compare the rates of disintegration, gastric emptying and drug absorption following administration of new and conventional paracetamol formulations. Kelly et al. conclude "it would seem that a combination of faster disintegration and gastric emptying of the new tablets [a combination of paracetamol and sodium bicarbonate] is responsible for the faster rate of absorption of paracetamol from such tablets".

Sterbenz et al., GB2103087A, disclose that, as measured by the time it takes after ingestion for the level of APAP to reach its maximum in the blood plasma of the subject to which it was administered (tₘₐₓ), the rate of absorption of APAP into the bloodstream can be increased by coadministering the APAP with about 60 mg to about 1200 mg, preferably about 400 mg to about 1000 mg, optimally about 450 mg to 880 mg of antacid. The weight of antacid used depends on its milliequivalent weight. Sterbenz et al. reference Wojcicki et al., Zbl Pharm. 118 (1979) Vol. 2-3, who found that when 4 grams of calcium carbonate were administered with 1 gram of APAP there was a significant decrease in the rate of absorption as measure by tₘₐₓ. The tₘₐₓ for APAP alone was 1.4 hours. The tₘₐₓ for calcium carbonate was 1.9 hours. In other words, the time it took for the plasma level of APAP to reach its maximum was 1/2 hour longer when the APAP was co-administered with the calcium carbonate than when it was administered alone. Sterbenz et al. state that when APAP is administered with the antacids at the levels called for by their invention, contrary to the teaching of Wojcicki et al., the tₘₐₓ values are lower when APAP and antacid are co-administered than when APAP is administered without the antacid.

In US patent 4.294.819. capsules containing acetylsalicylic acid and optionally other active analgesic materials in combination with an alkaline material are disclosed.

In DE 195 02 789 A1, multi-functional combinations attempting to treat all of the various symptoms of a "hangover" (alcohol abuse) with only one preparation are disclosed, for example oral dosage forms comprising acetylsalicylic acid, acetaminophen and sodium bicarbonate or acetylsalicylic acid and calcium/magnesium carbonate.

In US 6,316.025. tablets and capsules comprising acetaminophen and sodium bicarbonate are disclosed.

In US 5,296,241, suspensions for oral treatment of "hangover" (alcohol abuse) are disclosed, among them such comprising acetaminophen, caffeine and calcium carbonate.

### Summary of the Invention

Bristol-Myers Squibb Company markets EXCEDRIN, including EXCEDRIN MIGRAINE (under a Food and Drug Administration (FDA) approved New Drug Application (NDA)), EXCEDRIN EXTRA STRENGH and EXCEDRIN TENSION HEADACHE. EXCEDRIN MIGRAINE tablets contain aspirin, acetaminophen and caffeine. The present inventors postulated that enhancement of the absorption of the analgesics contained in the respective tablets, for example, EXCEDRIN MIGRAINE tablets, would produce a faster onset of the analgesic/antipyretic effect produced by the tablets.

In one embodiment, the present invention produces a stable analgesic/antipyretic dosage form, for example a tablet or caplet, having enhanced absorption of the analgesic/antipyretic active(s) contained therein and faster onset of analgesic/antipyretic activity.

In a further embodiment, the invention produces a stable analgesic/antipyretic dosage form, for example a tablet or caplet, such dosage form having enhanced absorption of the analgesic/antipyretic active(s) contained therein and faster onset of analgesic/antipyretic activity while maintaining bioequivalence with the respective tablets, for example EXCEDRIN MIGRAINE tablets.

In another embodiment of the invention, an improved aspirin, acetaminophen and caffeine containing dosage form was formulated that affords faster absorption of the analgesic/antipyretic actives, as compared to an FDA approved tablet, for example, the EXCEDRIN MIGRAINE tablet, while simultaneously keeping the peak blood level (Cₘₐₓ) and AUC parameters equivalent to that of the FDA approved tablets, thereby obtaining an improved faster acting product enabling suffers to obtain quicker relief.

The present inventors have surprisingly found that the inclusion of a buffer/alkaline species, for example, a carbonate, a bicarbonate or a mixture thereof, optionally with a pharmaceutically acceptable magnesium salt, in formulations containing analgesic/antipyretic actives, such as, aspirin, acetaminophen, and combinations of aspirin and/or acetaminophen, with caffeine, enhances the dissolution rate, speeds gastric emptying time, and possibly stimulates the gastro-intestinal tract, so that the analgesic/antipyretic actives are more rapidly absorbed and the onset of the analgesic/antipyretic effect is shortened.

### Detailed Description of the Invention

The composition of the present invention provides an analgesic/antipyretic composition comprising an analgesic/antipyretic effective amount of acetylsalicylic acid, acetaminophen and caffeine and an analgesic/antipyretic onset of analgesic/antipyretic activity shortening amount of at least one alkaline agent selected from the group consisting of calcium carbonate, a mixture of calcium carbonate and magnesium hydroxide and a mixture of calcium carbonate, magnesium carbonate and magnesium oxide.

Further embodiments of the invention are disclosed in the claims.

In one embodiment, the alkaline agent is calcium carbonate or a mixture of calcium carbonate and magnesium hydroxide

In yet another embodiment, the alkaline agent is a mixture of calcium carbonate and magnesium hydroxide.

The unit dosage form of the present invention can be a capsule (in which the alkaline agent(s) is (are) prevented from reacting with the acidic analgesic/antipyretic component(s) of the composition), a tablet or a caplet.

The unit dosage form of the present invention can also be a powder (or even an effervescent powder) which can be packaged in, for example a double pouch, in order to separate the acidic analgesic/antipyretic component(s) and alkaline agent(s).

The alkaline agent(s) can be contained in one tablet or caplet and the acidic analgesic/antipyretic component(s) can be contained in a separate tablet or caplet. The subject, generally a human, would be instructed to simultaneously or sequentially take one of each tablet or caplet.

In one embodiment, the dosage form is a tablet or caplet. In another embodiment, the dosage form is a multi-layer tablet or caplet In yet another embodiment, the dosage form is a two-layer tablet or caplet.

Another means of keeping the acidic analgesic/antipyretic component(s) of the composition from interacting with the alkaline agent(s) is by placing the acidic analgesic/antipyretic component(s) in a capsule and inserting the capsule into another capsule containing the alkaline agent(s). Alternatively, the alkaline agent(s) can be placed in a capsule and the capsule can be inserted into another capsule containing the acidic analgesic/antipyretic component(s). The caffeine can be present in either the inner or outer capsule or apportioned between both capsules. Instead of a capsule within another capsule, the dosage form can be a tablet within a capsule or a tablet compressed within another tablet.

Yet another means of keeping the acidic analgesic/antipyretic component(s) of the composition from interacting with the alkaline agent(s) is by coating the alkaline agent(s), the acidic analgesic/antipyretic component(s) or both, so as to create a physical barrier that prevents their interaction.

The aspirin and acetaminophen are kept physically separate from the alkaline agent(s) for reasons of stability. Thus, in a further embodiment, the aspirin and acetaminophen are formulated in one layer of a multi-layer tablet or caplet and the alkaline agent(s) is (are) formulated in another layer of the multi-layer tablet or caplet. The caffeine can be formulated in either layer or divided between both layers.

The composition employed to produce the tablet or caplet dosage form of the present invention may contain other materials typically employed in such formulations, for example, excipients such as starch, dextrose, sucrose or other saccharides, sorbitol, manitol, iso-malitol, or other compressible sugar alcohols, or mixtures thereof.

In one embodiment, the composition of the present invention will also contain citric acid, sodium phosphate and starch.

The present invention will now be elaborated upon with reference to the Examples which follow and the figures in which:
Figure 1 is a graph of the plasma salicylic acid concentration, as a function of time, for Examples 1 - 4;
Figure 2 is a graph of the plasma acetaminophen concentration, as a function of time, for Examples 1 - 4;
Figure 3 is a bar graph showing, for each time point, the truncated AUC values of salicylic acid for the compositions of Examples 1 - 4; and

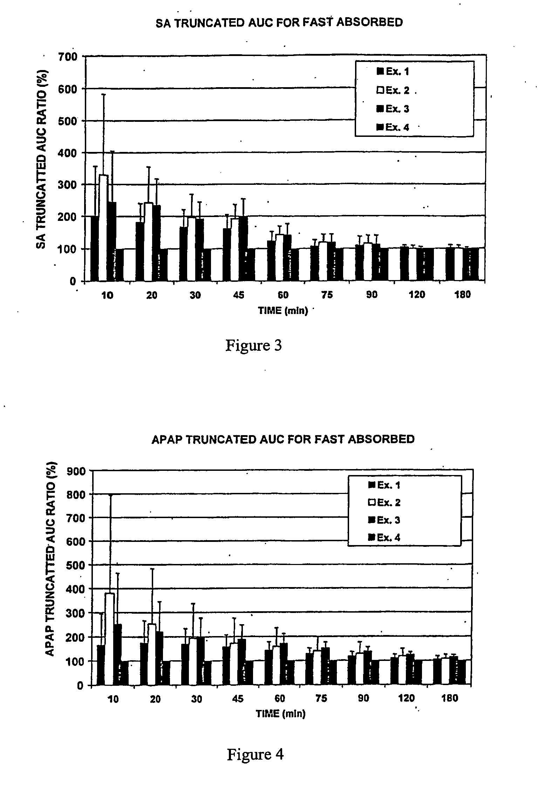

Figure 3 is a bar graph showing, for each time point, the truncated AUC values of acetaminophen for the compositions of Examples 1 - 4.

Compositions, in accordance with the present invention, and unit dosage forms formulated therefrom, may be produced by means of pharmaceutical processing techniques well known to those skilled in the art. The acetaminophen, caffeine and, when present, the aspirin, can be formulated into tablets or caplets by means of a dry mix/direct compression approach. The alkaline agent(s) can be formulated into the tablets/caplets by means of either a dry mix/direct compression approach or a wet granulation approach.

To prevent undesirable interaction of the acetaminophen, caffeine, aspirin (when present), the alkaline agent(s) and/or excipients, the unit dosage form can be formulated, for example, as a single, bi- or triple layer tablet/caplet.

In one embodiment, a wet granulation approach can be employed utilizing a mixer or granulator, for example, a Planetary mixer, high shear granulator or fluid bed granulator. The various processing techniques would require different formula compositions and/or different processing parameters that would be readily known or easily ascertainable by those skilled in the art.

In another embodiment, a dry mix process can be employed. In this embodiment, mixing equipment, such as, a V-blender (with or without an intensifier bar), a double-con blender, a Sigma-blade mixer, and a Tote blender, can be employed.

The embodiments which follow serve to illustrate processes that are employed in the manufacture of tablet/caplet unit dosage forms of the present invention.

In an embodiment, employing wet granulation and a high shear granulator, cold water is charged into a jacketed kettle equipped with a stirrer. Corn starch is dispersed into the cold water, and the resultant dispersion is heated in the range of 88-92°C while mixing to provide a starch paste. The resultant starch paste is cooled to a temperature of 45 - 50°C. In a separate a PMA 300 High Shear granulator, the alkaline agent(s) and caffeine are mixed for three (3) minutes at 150 RPM with the chopper at high position. After this time, the chopper is turned off and the starch paste is gradually added to the premix in the PMA 300 High Shear granulator over a period of about ten (10) minutes. The resultant wet granulation is passed through a Comil equipped with a 500 Q screen using a round blade. The screened wet granulation is then dried in a Glatt Fluid Bed dryer, with inlet air set at 70° C and air volume at 1200 CFM, until the product temperature reaches about 42° - 46°C and the moisture content, as determined with the aid of a Mettler (program 3, temperature 105° C, using ∼5 grams of sample) or Computrac Moisture Balance, is in the range of 1.2 - 2.5%. Once at the prescribed temperature and moisture content, the dry granulation is milled with the aid of a Comil (equipped with screen 075R and a round blade). After this time, the milled granulation is charged through a 10 mesh screen into a Tote blender (or V-blender) and then mixed with the excipients for five (5) minutes. At the conclusion of this period, the lubricant is charged into the blender through a 30 mesh screen, and the resulting mixture is mixed for three (3) minutes to produce the desired product.

In another embodiment, a fluid bed granulator is employed as follows:
The chamber of a Glatt Fluid Bed granulator is pre-heated to 70 °C by setting the inlet air temperature to 70 °C and the air flap at 30-35%. Once at the prescribed temperature, a mix of the caffeine and alkaline agent(s) is charged into the Glatt bowl, and then, while heating the powder mix, the starch paste is sprayed into to the mix at a rate of 130-180 g/min. The granulation process is periodically checked for consistency and the spray rate and inlet air temperature are adjusted, as needed, to ensure production of a good granulation. The resulting granulation is dried in the manner set forth above and then milled at a speed of 900 rpm with the aid of a Comil, equipped with screen No. 094R.
The milled dried granulation is then charged into a two (2) cubic foot Tote blender and the required amount of lubricant (previously screened through # 30 mesh) is charged into the blender. The resultant mix is blended for five (5) minutes at 24 rpm to achieve the desired product.

In another embodiment, a dry mix method is employed as follows:
(i) The caffeine, alkaline agent(s) and excipients are sequentially charged into a five (5) cubic foot Tote blender and mixed for twenty (20) minutes. After this time, the lubricant is screened through #30 mesh and then charged into the blender. The resultant mix is blended for five (5) minutes at 12 rpm to afford a caffeine/alkaline agent(s) blend.
(ii) The acetaminophen, aspirin and excipients are sequentially charged into a separate five (5) cubic foot Tote blender and mixed for twenty (20) minutes.
   The lubricant is added in the manner set forth in (i) above, and the resultant mix is blended for five (5) minutes at 12 rpm to afford an acetaminophen/aspirin blend.

Caplets (or tablets) are produced utilizing the blends produced in (i) and (ii) above as follows:
The acetaminophen/aspirin blend is charged into one hopper of a Two-Layer Kikusui tablet press fitted with 0.290" x 0.725" caplet shape tooling (or when tablets are desired, the appropriate tablet shape tooling). The caffeine/alkaline agent(s) blend is charged into the other hopper. In one embodiment, the caplets (or tablets) are produced in accordance with the following specifications:

| | **Target** | **Action Limits** |
|---|---|---|
| **Weight (mg)** | 777.2 mg | 757.2-797.2 mg |
| **Hardness (SCU)** | 16 SCU | 14-18 SCU |
| **Thickness (inches)** | 0.243" | 0.240-0.246" |
| ***Main compression force(KN)*** | 14 KN | 15 - 17 KN |
| ***Pre-compression force (KN)*** | *1.0 KN* | *0. 9 - 1.1 KN* |

The tablet and caplet dosage forms of the present invention are optionally coated with an aqueous or solvent film coating using methods and equipment, for example, a perforated coating pan or a Fluid Bed with Wurster Column, commonly used by those skilled in the art.

In one embodiment, the film coating suspension is applied onto cores in a perforated coating pan while maintaining the following conditions:
Pan load: 12 Kg
Inlet air temperature: 50-65°C
Exhaust temperature: 40-45°C
Air volume: 300-350 CFM
Spray rate: 40-60 g/minute
Pan speed: 8-15 rpm

The coated caplets (or tablets) are then cooled to 25-30°C. Optionally, the exhaust blower is bypassed and carnauba wax is applied to the coated caplets (or tablets) to polish their surfaces.

The following Examples are offered to illustrate the present invention

**Examples 1 - 4**

| Analgesic/Antipyretic Compositions | | | | |
|---|---|---|---|---|
| Ingredient | Ex. 1 | Ex. 2 | Ex. 3 | Ex. 4 |
| Aspirin | 250 mg | 250 mg | 250 mg | 250 mg |
| Acetaminophen | 250 mg | 250 mg | 250 mg | 250 mg |
| Caffeine | 65 mg | 65 mg | 65 mg | 65 mg |
| Calcium carbonate | 150 mg | 43 mg | 180 mg | - |
| Magnesium hydroxide | - | 75 mg | - | |
| Magnesium oxide | - | - | 45 mg | - |
| Magnesium carbonate | - | - | 63mg | - |
| Citric acid | 5 mg | 5 mg | 10 mg | 5 mg |
| Sodium phosphate | 5 mg | 5 mg | 5 mg | 5 mg |
| Starch | q.s | q.s | q.s. | q.s |

It should be noted that Examples 1 - 3 are illustrative of the fast acting compositions of the present invention. Example 4 is a like composition but without the alkaline agent called for by the present invention.

A preclinical pharmacokinetic (PK) study was carried out utilizing tablets prepared from the compositions of Examples 1 - 4.

A single-dose, four-sequence, four-period, four formulation, crossover Williams Design was used to differentiate the pharmacokinetic behaviors between the compositions of Examples 1 - 3 and that of Example 4. Each sequence contained two (2) naive beagle dogs. The washout period was one week. Serial blood samples were collected for a period of twelve (12) hours after oral administration. The plasma concentrations of caffeine, acetaminophen, and salicylic acid were determined using a validated HPLC method. One dog vomited in its second dosing and its' samples were not analyzed.

Pharmacokinetic values, including the time it takes for a drug to reach peak blood level (Tₘₐₓ) and truncated AUC, were determined by non-compartmental analysis. Analysis of variance (ANOVA) was performed on the means to determine statistical significance. The classical pharmacokinetic parameters, Cₘₐₓ and/or AUC, showed the compositions of Example 1 - 3 to be bioequivalent to the composition of Example 4.

In addition, a truncated AUC, reflective of plasma levels at early time points, was employed to reflect what happens during the time intervals of interest (ten (10) minute (T10), twenty (20) minutes (T20), and thirty (30) minutes (T30)), and indicate a faster onset of activity.

The following conclusions can be drawn from the study results:
(1) Table 1, which follows, shows the plasma level of salicylic acid in each dog in the study.

**Table 1**

| The plasma concentrations of salicylic acid (ng/ml) 10, 20 and 30 minutes post dosing. | | | | | | | | | | | |
|---|---|---|---|---|---|---|---|---|---|---|---|
| Conc. | | | | | | | | | | | |
| SA | | | | | | | | | | | |
| A | H00473 | H00479 | H00475 | H00480 | H00478 | H00481 | H00474 | HO0476 | AVE | SD | RSD |
| 10min | 22800 | 38100 | 17300 | 5120 | 12300 | 6130 | 9900 | 15000 | 15831 | 10716 | 68 |
| 20min | 52300 | 41700 | 57800 | 31200 | 64400 | 57100 | 60100 | 24900 | 46900 | 28400 | 38 |
| 30min | 59200 | 57400 | 66100 | 36400 | 75400 | 76500 | 44500 | 30500 | 60000 | 45100 | 26 |
| | | | | | | | | | | | |
| B | | | | | | | | | | | |
| 10 min | 18400 | | 27800 | 18600 | 11100 | 26300 | 31300 | 12800 | 20900 | 7726 | 37 |
| 20min | 41700 | | 45000 | 44600 | 30600 | 47300 | 56400 | 46000 | 44514 | 7670 | 17 |
| 30min | 57400 | | 54000 | 61900 | 38000 | 58200 | 67600 | 58500 | 56514 | 9207 | 16 |
| | | | | | | | | | | | |
| C | | | | | | | | | | | |
| 10min | 27300 | 30400 | 24600 | 14400 | 16500 | 12000 | 11500 | 8660 | 18170 | 8144 | 45 |
| 20min | 57800 | 60100 | 46900 | 42200 | 47500 | 38000 | 41200 | 35000 | 46088 | 8972 | 19 |
| 30min | 66100 | 44500 | 60000 | 52500 | 60900 | 54000 | 59200 | 55200 | 56550 | 6535 | 12 |
| | | | | | | | | | | | |
| D | | | | | | | | | | | |
| 10min | 16100 | 7620 | 4470 | 7720 | 15600 | 4150 | 6080 | 12100 | 9230 | 4770 | 52 |
| 20min | 31200 | 24900 | 12100 | 20000 | 23900 | 12600 | 17300 | 31500 | 21688 | 7546 | 35 |
| 30min | 36400 | 30500 | 42000 | 27100 | 28600 | 18700 | 25100 | 40600 | 31125 | 8014 | 26 |

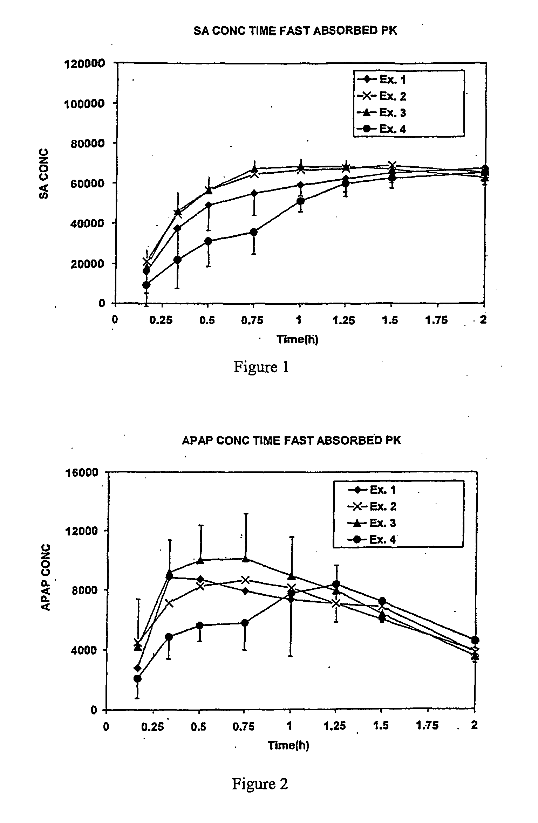

In Table 1, above, A, B, C, and D respectively represent the compositions of Examples 1, 2, 3 and 4 and HO0473 to HO0476 represent dog numbers.
The results of Table 1 demonstrate that with the compositions of Examples 1 - 3 there was a faster uptake of salicylic acid in the plasma during the early time intervals post dose (10 minutes to 30 minutes), as compared to the composition of Example 4 (a like composition but without alkaline agent).
The average plasma level of salicylic acid as a function of time is shown in Figure 1.
As shown in Table 1 and Figure 1, the average level of salicylic acid in the plasma at T10 after administration of the compositions of Examples 1, 2 and 3 was 15834 ± 10716 ng/ml (Example 1), 20900 ± 7726 ng/ml (Example 2), 18170 ± 8144 ng/ml (Example 3) compared to 9230 ± 4770 ng/ml (Example 4).
In other words, with the same level of aspirin being delivered from the three different compositions of the present invention, at T10, there was about 1.5∼2 times more salicylic acid present in the plasma than was present after administration of the composition of Example 4.
An ANOVA analysis showed that plasma level at T10 from the compositions of Examples 1, 2 and 3 is statistically greater than the plasma level at T10 from the composition of Example 4 (p=0.05).
As the study was crossover in design, the plasma level difference in each individual dog could be examined. It clearly showed that, as compared to dogs dosed with the composition of Example 4, 6 out of 8 dogs had a 1.5 ∼ 5.5 times higher plasma salicylic acid level at T10 when dosed with the composition of Example 3. Only one dog was slightly lower.
(2) During the early time intervals post dose (10 minutes to 30 minutes post dosing), the plasma uptake of acetaminophen was faster with the compositions of Examples 1 - 3 than with the composition of Example 4.
Figure 2 shows the average plasma level of acetaminophen and Table 2, below, shows the plasma level of acetaminophen in each dog in the study.
As shown in Table 2 and Figure 2, the average plasma acetaminophen level at T10 after administration of the test compositions, was 2759 ± 2971 ng/ml (Example 1), 4461 ± 3383 ng/ml (Example 2), 4173 ± 3193 ng/ml (Example 3) and 2071 ± 1307 ng/ml (Example 4). In other words, although the same amount of acetaminophen was delivered in all of the test compositions, with the compositions of the present invention (Examples 1, 2 and 3) at T10 there was, with the compositions of Examples 2 and 3, about 2 times more acetaminophen present in the plasma (an over 200% increase), and with the composition of Example 1, an over 33% increase, as compared with the results obtained with the composition of Example 4.
As shown in Table 2, 7 out of 8 dogs had a 1.3 ∼ 3.0 times higher acetaminophen plasma level at T20 with composition A (Example 1) than with composition D, with only one dog slightly lower.

**Table 2.**

| The plasma concentrations of acetaminophen (ng/ml) 10,20 and 30 minutes post dosing. | | | | | | | | | | | |
|---|---|---|---|---|---|---|---|---|---|---|---|
| Conc. | | | | | | | | | | | |
| APAP | | | | | | | | | | | |
| A | H00473 | H00479 | H00475 | H00480 | H00478 | H00481 | H00474 | H00476 | AVE | SD | RSD |
| 10min | 3950 | 9600 | 2800 | 500 | 1260 | 1350 | 1260 | 1350 | 2759 | 2971 | 108 |
| 20min | 11700 | 13600 | 7730 | 3140 | 8700 | 8490 | 8700 | 8490 | 8819 | 3043 | 35 |
| 30min | 11500 | 13500 | 8440 | 5400 | 7210 | 8180 | 7210 | 8180 | 8703 | 2586 | 30 |
| | | | | | | | | | | | |
| B | | | | | | | | | | | |
| 10min | 1560 | | 8480 | 3100 | 1230 | 6020 | 9130 | 1710 | 4461 | 3383 | 76 |
| 20min | 5260 | | 14600 | 4910 | 4230 | 6090 | 8810 | 5710 | 7087 | 3618 | 51 |
| 30min | 8230 | | 14500 | 7790 | 4100 | 7660 | 10200 | 5100 | 8226 | 3429 | 42 |
| | | | | | | | | | | | |
| C | | | | | | | | | | | |
| 10min | 4850 | 11100 | 5310 | 1780 | 4270 | 2880 | 2280 | 910 | 4173 | 3193 | 77 |
| 20min | 9570 | 13700 | 10700 | 6760 | 8510 | 7490 | 8240 | 8300 | 9159 | 2194 | 24 |
| 30min | 10900 | 11800 | 12700 | 5660 | 8160 | 9140 | 12100 | 9550 | 10001 | 2358 | 24 |
| | | | | | | | | | | | |
| D | | | | | | | | | | | |
| 10min | 3840 | 2430 | 889 | 1280 | 4140 | 645 | 1870 | 1470 | 2071 | 1309 | 63 |
| 20min | 6650 | 6350 | 3050 | 4110 | 4850 | 2800 | 4740 | 6120 | 4834 | 1469 | 30 |
| 30min | 6640 | 5860 | 5360 | 4710 | 4760 | 4340 | 5670 | 7450 | 5599 | 1050 | 19 |

In Table 2, above, A, B, C, and D respectively represent the compositions of Examples 1, 2, 3 and 4 and HO0473 to HO0476 represent dog numbers.
(3) During the early time intervals post dose: (ten (10) to thirty (30) minutes post dosing), the compositions of Examples 1, 2 and 3, produced a higher truncated AUC of salicylic acid than was produced by the composition of Example 4.
Table 3, below, shows the truncated AUC values of salicylic acid for each dog in the study.
As shown in Table 3, at T10 after administration, the average level of truncated AUC of salicylic acid for the compositions of Examples 1- 4, was 1322 ± 895 ng/ml*hr (Example 1), 1745 ± 645 ng/ml*hr (Example 2), 1517 ± 680 ng/ml*hr (Example 3) and 771 ± 398 ng/ml*hr (Example 4), respectively.
Figure 3 graphically illustrates, at each time point, the truncated AUCs of the compositions of Examples 1 - 4. At the final time point (180 minutes (T180) post dosing) the ratios of truncated AUC for salicylic acid (SA) for all compositions (Examples 1, 2, 3 and 4) were close to 100%. This indicates that the compositions of Examples 1, 2 and 3, in accordance with the present invention, were AUC bioequivalent to the composition of Example 4.
Surprisingly, although the compositions of the present invention were bioequivalent to a like composition that does not contain alkaline agent, at the early time points, the compositions of Examples 1, 2 and 3 were more than three (3) times higher in truncated AUC for salicylic acid than the composition of Example 4.

**Table 3**

| The truncated AUC values of salicylic acid (ng/ml*hr) 10, 20 and 30 min post dosing. | | | | | | | | | | | |
|---|---|---|---|---|---|---|---|---|---|---|---|
| AUC | | | | | | | | | | | |
| SA | | | | | | | | | | | |
| A | HO0473 | H00479 | H00475 | H00480 | HO0478 | H00481 | H00474 | HO0476 | AVE | SD | RSD |
| 10min | 1904 | 3181 | 1445 | 428 | 1027 | 512 | 827 | 1253 | 1322 | 895 | 68 |
| 20min | 8025 | 11535 | 5170 | 2565 | 5232 | 3481 | 4071 | 5149 | 5654 | 2870 | 51 |
| 30min | 17503 | 23418 | 11418 | 7572 | 12610 | 9984 | 9928 | 11490 | 12990 | 5095 | 39 |
| | | | | | | | | | | | |
| B | | | | | | | | | | | |
| 10min | 1536 | | 2321 | 1553 | 927 | 2196 | 2614 | 1069 | 1745 | 645 | 37 |
| 20min | 6434 | | 8254 | 6704 | 4326 | 8194 | 9762 | 5861 | 7076 | 1800 | 25 |
| 30min | 14858 | | 16669 | 15757 | 10157 | 17162 | 20302 | 14744 | 15664 | 3071 | 20 |
| | | | | | | | | | | | |
| C | | | | | | | | | | | |
| 10min | 2280 | 2538 | 2054 | 1202 | 1378 | 1002 | 960 | 723 | 1517 | 680 | 45 |
| 20min | 9216 | 9914 | 7881 | 5815 | 6594 | 5077 | 5255 | 4281 | 6754 | 2049 | 030 |
| 30min | 19748 | 18805 | 16968 | 13865 | 15808 | 12897 | 13789 | 11948 | 15479 | 2834 | 018 |
| | | | | | | | | | | | |
| D 10min | 1344 | 636 | 373 | 645 | 1303 | 347 | 508 | 1010 | 771 | 398 | 52 |
| 20min | 5199 | 3286 | 1723 | 2904 | 4522 | 1712 | 2413 | 4563 | 3290 | 1344 | 41 |
| 30min | 10945 | 7995 | 6322 | 6908 | 8985 | 4373 | 6017 | 10692 | 7780 | 2318 | 30 |

In Table 3, above, A, B, C, and D respectively represent the compositions of Examples 1, 2, 3 and 4 and HO0473 to HO0476 represent dog numbers.
(4) The compositions of Examples 1, 2 and 3 exhibited a higher truncated AUC of acetaminophen during the 10 minute, 20 minute and 30 minute post dose periods, as compared to the composition of Example 4 (a like composition but not containing alkaline agent).
Table 4, which follows, shows the truncated AUC values of acetaminophen in each dog in the study. As shown in Table 4, at T20 after administration, the average level of truncated AUC of acetaminophen present after administration of the compositions of Examples 1, 2 and 3 was 1174 ± 703 ng/ml*hr (Example 1), 1283 ± 833 ng/ml*hr (Example 2), 1422 ± 711 ng/ml*hr (Example 3) compared to 728 ± 314 ng/ml*hr after administration of the composition of Example 4..
Figure 4 graphically shows, for each of Examples 1 - 4, the truncated AUCs of acetaminophen at each time point. At the final point (T180) the ratios of truncated AUC for acetaminophen for the compositions of Examples 1, 2, 3 and 4 were close to 100%, indicating the compositions of Examples 1, 2 and 3 are all AUC bioequivalent to the composition of Example 4. However, at the early time points, the compositions of Examples 1, 2 and 3, in accordance with the present invention, exhibited a truncated AUC of acetaminophen more than three (3) times greater than that exhibited by the composition of Example 4.

**Table 4**

| The truncated AUC values of acetaminophen (ng/ml*hr) at the 10, 20 and 30 minute time points. | | | | | | | | | | | |
|---|---|---|---|---|---|---|---|---|---|---|---|
| AUC | | | | | | | | | | | |
| APAP | | | | | | | | | | | |
| A | H00473 | H00479 | H00475 | H00480 | H00478 | H00481 | H00474 | H00476 | AVE | SD | RSD |
| 10min | 330 | 802 | 234 | 42 | 105 | 113 | 105 | 113 | 231 | 248 | 10 |
| 20min | 1605 | 2693 | 1092 | 339 | 917 | 915 | 917 | 915 | 1174 | 703 | 60 |
| 30min | 3577 | 4997 | 2466 | 1065 | 2269 | 2332 | 2269 | 2332 | 2663 | 1159 | 44 |
| | | | | | | | | | | | |
| B | | | | | | | | | | | |
| 10min | 130 | | 708 | 42 | 103 | 503 | 762 | 143 | 342 | 308 | 90 |
| 20min | 686 | | 2589 | 695 | 548 | 1490 | 2224 | 748 | 1283 | 833 | 65 |
| 30min | 1833 | | 5063 | 1775 | 1256 | 2659 | 3840 | 1667 | 2585 | 1388 | 54 |
| | | | | | | | | | | | |
| C | | | | | | | | | | | |
| 10min | 405 | 927 | 443 | 042 | 357 | 240 | 190 | 076 | 335 | 280 | 084 |
| 20min | 1580 | 2948 | 1748 | 738 | 1399 | 1085 | 1047 | 827 | 1422 | 711 | 050 |
| 30min | 3320 | 5116 | 3737 | 1794 | 2816 | 2499 | 2776 | 2344 | 3050 | 1023 | 034 |
| | | | | | | | | | | | |
| D | | | | | | | | | | | |
| 10min | 321 | 203 | 74 | 42 | 346 | 54 | 156 | 123 | 165 | 117 | 71 |
| 20min | 1176 | 919 | 395 | 481 | 1079 | 335 | 695 | 742 | 728 | 314 | 43 |
| 30min | 2306 | 1957 | 1110 | 1231 | 1896 | 942 | 1580 | 1895 | 1615 | 479 | 30 |

In Table 4, above, A, B, C, and D respectively represent the compositions of Examples 1, 2, 3 and 4 and HO0473 to HO0476 represent dog numbers.
(5) The median (mean) Tₘₐₓ (acetaminophen) for Examples 1, 2, 3 and 4 were 0.50 (0.78), 0.75 (0.74), 0.50 (0.56), and 1.25 (1.29) hours, respectively.

The ANOVA model, based on the ranked Tₘₐₓ values, indicated that Tₘₐₓ (acetaminophen) for the compositions of Examples 1, 2 and 3 was statistically significantly lower than for the composition of Example 4.

The median (mean) Tₘₐₓ (salicylic acid) for the compositions of Examples 1, 2, 3 and 4 were 1.50 (1.68),1.25 (1.25),1.25 (1.21), and 1.50 (1.68) hours, respectively.

The ANOVA model based on the ranked Tₘₐₓ values (salicylic acid) indicated no statistically significant difference between the compositions of Example 1 and Example 2. However, the Tₘₐₓ (salicylic acid) for the compositions of Examples 2 and 3 was statistically significantly lower than the Tₘₐₓ (salicylic acid) for the composition of Example 4.

This data indicates that the compositions of Examples 1, 2 and 3, in accordance with the present invention, provided a faster onset of analgesic/antipyretic activity than the composition of Example 4.

## Claims

1. An analgesic/antipyretic composition comprising an analgesic/antipyretic effective amount of acetylsalicylic acid, acetaminophen and caffeine and an analgesic/antipyretic onset of analgesic/antipyretic activity shortening amount of at least one alkaline agent selected from the group consisting of calcium carbonate, a mixture of calcium carbonate and magnesium hydroxide and a mixture of calcium carbonate, magnesium carbonate and magnesium oxide.

2. An analgesic/antipyretic composition according to claim 1 comprised of acetylsalicylic acid, acetaminophen, caffeine, calcium carbonate, citric acid, sodium phosphate, and starch, said analgesic/antipyretic composition having enhanced absorption of the acetylsalicylic acid and acetaminophen contained therein and faster onset of analgesic/antipyretic activity.

3. An analgesic/antipyretic composition according to claim 2, further comprising magnesium hydroxide.

4. An analgesic/antipyretic composition according to claim 3, wherein the composition contains 250 mg of acetylsalicylic acid, 250 mg of acetaminophen, 65 mg of caffeine, 43 mg of calcium carbonate, 75 mg of magnesium hydroxide, 5 mg of citric acid, and 5 mg of sodium phosphate.

5. An analgesic/antipyretic unit dosage form comprising 25 mg to 2.5 g acetylsalicylic acid, 25 mg to 2 g acetaminophen, 5 mg to 500 mg caffeine and 25 mg to 2.5 g of at least one alkaline agent.

6. An analgesic/antipyretic unit dosage form according to claim 5 comprising 81 mg to 1 g acetylsalicylic acid, 100 mg to 1 g acetaminophen, 15 mg to 250 mg caffeine and 50 mg to 1 g of at least one alkaline agent.

7. An analgesic/antipyretic unit dosage form according to claim 5 comprising 150 mg to 500 mg acetylsalicylic acid, 150 mg to 500 mg acetaminophen, 30 mg to 150 mg caffeine and 75 mg to a 500 mg of at least one alkaline agent.

8. An analgesic/antipyretic unit dosage form according to claim 5 comprising 250 mg acetylsalicylic acid, 250 mg acetaminophen. 65 mg caffeine and 100 mg to 300 mg of at least one alkaline agent.

9. A unit dosage form according to any one of claims 5-8, wherein the at least one alkaline agent is selected from the group consisting of calcium carbonate and a mixture of calcium carbonate and magnesium hydroxide.

## Patentansprüche

1. Analyetische/antipyretische Zusammensetzung umfassend eine analgetisch/antipyretisch wirksame Menge von Acetylsalicylsäure, Acetaminophen und Koffein und einen analgetischen/antipyretischen Beginn der analgetischen/antipyretischen Aktivität, die die Menge mindestens eines alkalischen Mittels, ausgewählt aus der Gruppe bestehend aus Calciumkarbonat, einer Mischung aus Calciumkarbonat und Magnesiumhydroxid und einer Mischung aus Calciumkarbonat, Magnesiumkarbonat und Magnesiumoxid, verringert

2. Analgetische/antipyretische Zusammensetzung nach Anspruch 1, die Acetylsalicylsäure, Acetaminophen, Koffein, Calciumkarbonat, Zitronensäure, Natriumphosphat und Stärke umfasst, wobei die analgetische/antipyretische Zusammensetzung eine erhöhte Absorption der Acetylsalicylsäure und des Acetaminophens zeigt, die darin enthalten sind und einen schnelleren Beginn der analgetischen/antipyretischen Aktivität.

3. Analgetische/antipyretische Zusammensetzung nach Anspruch 2, weiterhin umfassend Magnesiumhydroxid.

4. Analgetische/antipyretische Zusammensetzung nach Anspruch 3, wobei die Zusammensetzung 250 mg Acetylsalicylsäure, 250 mg Acetaminophen, 65 mg Koffein, 43 mg Calciumkarbonat, 75 mg Magnesiumhydroxid, 5 mg Zitronensäure und 5 mg Natriumphosphat enthält.

5. Analgetische/antipyretische Einzeldosisform, umfassend 25 mg bis 2,5 g Acetylsalicylsäure, 25 mg bis 2 g Acetaminophen, 5 mg bis 500 mg Koffein und 25 mg bis 2,5 g mindestens eines alkalischen Mittels.

6. Analgetische/antipyretische Einzeldosisform nach Anspruch 5, umfassend 81 mg bis 1 g Acetylsalicylsäure, 100 mg bis 1 g Acetaminophen, 15 mg bis 250 mg Koffein und 50 mg bis 1 g mindestens eines alkalischen Mittels.

7. Analgetische/antipyretische Einzeldosisform nach Anspruch 5, umfassend 150 mg bis 500 mg Acetylsalicylsäure, 150 mg bis 500 mg Acetaminophen, 30 mg bis 150 mg Koffein und 75 mg bis 500 mg mindestens eines alkalischen Mittels.

8. Analgetische/antipyretische Einzeldosisform nach Anspruch 5, umfassend 250 mg Acetylsalicylsäure, 250 mg Acetaminophen, 65 mg Koffein und 100 mg bis 300 mg mindestens eines alkalischen Mittels.

9. Einzeldosisform nach einem der Ansprüche 5-8, wobei das mindestens eine alkalische Mittel ausgewählt ist aus der Gruppe bestehend aus Calciumkarbonat und einer Mischung aus Calciumkarbonat und Magnesiumhydroxid.

## Revendications

1. Composition analgésique/antipyrétique comprenant une quantité analgésique/antipyrétique efficace d'acide acétylsalicylique d'acétaminophène et de caféine et une mise en place analgésique/antipyrétique d'une activité analgésique/antipyrétique diminuant la quantité d'au moins un agent alcalin sélectionné à partir du groupe composé de carbonate de calcium, d'un mélange de carbonate de calcium et d'hydroxyde de magnésium et d'un mélange de carbonate de calcium, de carbonate de magnésium et d'oxyde de magnésium.

2. Composition analgésique/antipyrétique selon la revendication 1 composée d'acide acétylsalicylique, d'acétaminophéne, de caféine, de carbonate de calcium, d'acide citrique, de phosphate de sodium, et d'amidon, ladite composition analgésique/antipyrétique faisant preuve d'une absorption accrue de l'acide acétylsalicylique et de l'acétaminophène qui y sont contenus et d'une mise en palace plus rapide de l'activité analgésique/antipyrétique.

3. Composition anatgésique/antipyrétique sdon la revendication 2 comprenant également de l'hydroxyde de magnésium.

4. Composition analgésique/antipyrétique selon la revendication 3, **caractérisée en ce que** la composition contient 250 mg d'acide acétylsalicylique, 250 mg d'acétaminophène, 65 mg de caféine, 43 mg de carbonate de calcium, 75 mg d'hydroxyde de magnésium, 5 mg d'acide citrique, et 5 mg de phosphate de sodium.

5. Forme due dosage unitaire anlgésique/antipyrétique comprenant de 25 mg à 2,5 g d'acide acétylsalicylique, de 25 mg à 2 g d'acétaminophène, de 5 mg à 500 mg de caféine et de 25 mg à 2,5 g d'au moins un agent alcalin.

6. Forme de dosage unitaire analgésique/antipyrétique selon la revendication 5 comprenant de 81 mg à 1 g d'acide acétylsalicylique, de 100 mg à 1 g d'acétaminophèns, de 15 mg à 250 mg de caféine et de 50 mg à 1 g d'au moins un agent alcalin.

7. Forme de dosage unitaire anlgésique/antipyrétique selon la revendication 5 comprenant de 150 mg à 500 mg d'acide acétylsalicylique de 150 mg à 500 mg d'acétaminophène, de 30 mg à 150 mg de caféine et de 75 mg à 500 mg d'au moins un agent alcalin.

8. Forme de dosage unitaire analgésique/antipyétique selon la revendication 5 comprenant 250 mg d'acide acetylsalicylique, 250 mg d'acétaminophène, 65 mg de caféine et de 100 mg à 300 mg d'au moins un agent alcalin,

9. Forme de dosage unitaire analgésique/antipyrétique selon l'une quelconque des revendication 5-8, **caractérisée en ce que** l'agent alcalin est sélectionné à partir du groupe composé de carbonate de calcium et d'un mélange de carbonate de calcium et d'hydroxyde de magnésium.
